# EUROPÄISCHE PATENTSCHRIFT

(11) **EP 0 943 604 B1**
(45) Veröffentlichungstag und Bekanntmachung des Hinweises auf die Patenterteilung: **21.11.2001**
(21) Anmeldenummer: 99103873.8
(22) Anmeldetag: 01.03.1999
(51) Int. Cl.: C07C 253/00, C07C 255/04, C07C 255/64

(54) **Verfahren zur Herstellung von Malonsäuredinitril**
Process for the preparation of malononitrile
Procédé pour la préparation de malononitrile

(30) Priorität: 19.03.1998 CH 66498
(43) Veröffentlichungstag der Anmeldung: 22.09.1999
(73) Patentinhaber: LONZA A.G., CH-4002 Basel (CH)
(72) Erfinder: Chen, Peter, Prof. Dr., 8044 Zürich (CH); Hoffner, Johannes, Dr., 8053 Zürich (CH); Müller, André, 8954 Geroldswil (CH); Fuchs, Rudolf, Dr., 1950 Sion (CH)

(56) Entgegenhaltungen:
- WO-A-98/05630
- US-A- 2 553 406
- US-A- 2 606 917

## Beschreibung

Die Erfindung betrifft ein neues Verfahren zur Herstellung von Malonsäuredinitril.

Malonsäuredinitril ist ein Ausgangs- und Zwischenprodukt von zentraler Bedeutung für die Herstellung von einer ausserordentlichen Vielfalt von z. B. pharmazeutischen oder agrochemischen Wirkstoffen (Ullmann's Encyklopädie der technischen Chemie, 4. neub. und erw. Auflage, Verlag Chemie Weinheim, Band 16, S. 419-423).

Zur Herstellung von Malonsäuredinitril sind zahlreiche Verfahren bekannt, grosstechnische Bedeutung hat allerdings lediglich die Hochtemperaturumsetzung von Acetonitril mit Chlorcyan bei Temperaturen von über 700 °C erlangt.

Die Aufgabe der Erfindung bestand darin, alternative Verfahren mit Potential für den grosstechnischen Einsatz zu entwickeln.
Die Aufgabe konnte gelöst werden mit dem neuen Verfahren gemäss Patentanspruch 1.

Erfindungsgemäss wird ein 2-Cyano-*N*-alkoxy-acetimidoylhalogenid der allgemeinen Formel 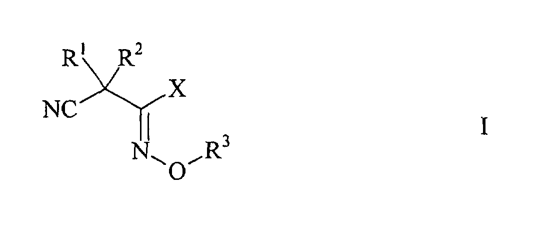 worin R¹ und R² gleich oder verschieden sind und Wasserstoff oder Alkyl bedeuten, R³ Alkyl, Cycloalkyl, Aryl, Arylalkyl oder eine Gruppe der Formel 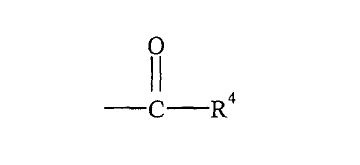 bedeutet, worin R⁴ für eine Alkyl, Aryl oder Arylalkylgruppe steht, und X ein Halogenatom bedeutet, bei einer Temperatur von 500 °C bis 1000 °C zu Malonsäuredinitril umgesetzt. Unter einer Alkylgruppe wird zweckmässig eine C₁₋₆-Alkylgruppe, namentlich Methyl, Ethyl, n-Propyl, i-Propyl, n-Butyl, i-Butyl, t-Butyl, Pentyl und seine Isomeren oder Hexyl und seine Isomeren verstanden. Bevorzugte Bedeutung von R¹ ist Methyl.
Cycloalkyl steht zweckmässig für eine C₃₋₆-Cycloalkylgruppe, namentlich Cyclopropyl, Cyclobutyl, Cyclopentyl oder Cyclohexyl.
Aryl steht zweckmässig für gegebenenfalls substituiertes Phenyl oder Naphthyl und Arylalkyl zweckmässig für eine Benzylgruppe.
Sowohl die Alkyl- als auch die Arylgruppe kann mit geeigneten Substituenten versehen sein. Als Beispiel seien genannt: C₁₋₄-Alkyl, C₁₋₄-Alkoxy, C₁₋₄-Alkanoyl, Halogen, Nitro, Amino, Alkylamino oder Dialkylamino.

Halogen hat die Bedeutung von Fluor, Chlor, Brom oder Jod, bevorzugt von Brom oder Chlor. Die 2-Cyano-*N*-alkoxy-acetimidoylhalogenide, als Ausgangsverbindung für die Hochtemperaturbehandlung, lassen sich zweckmässig durch Halogenierung eines 2-Cyano-*N*-alkoxy-acetamids der allgemeinen Formel 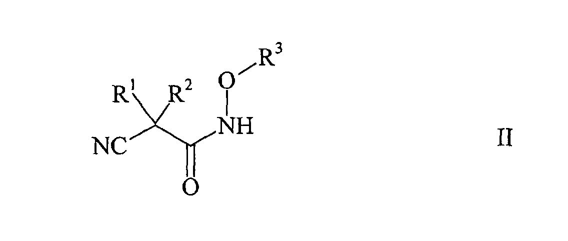 worin R¹, R² und R³ die genannten Bedeutungen haben, herstellen.

Die Halogenierung ist vorzugsweise eine Chlorierung und wird mit geeigneten Halogenierungsmitteln wie z. B. Phosphorpentachlorid, Phosgen, Phosphoroxychlorid oder Tetrachlorkohlenstoff und Triphenylphosphin durchgeführt.
Zweckmässig wird in einem geeigneten Lösungsmittel, vorzugsweise einem halogenierten Lösungsmittel wie z.B. Chloroform oder Methylenchlorid gearbeitet.
Die Reaktionstemperatur für die Halogenierung beträgt zweckmässig -20 °C bis 150 °C. Das entsprechende 2-Cyano-*N*-alkoxy-acetimidoylhalogenid kann auf fachmännische Weise aus dem Reaktionsgemisch, z. B. durch Extraktion, erhalten und nach Entfernung des Lösungsmittels für die Weiterumsetzung verwendet werden.

Die erfindungsgemässe Hochtemperaturumsetzung verläuft vorzugsweise bei einer Temperatur von 700 °C bis 1000 °C. Üblicherweise wird die Reaktion in einem Rohrreaktor durchgeführt. Die Umsetzungszeit beträgt in der Regel wenige Sekunden.
Von Vorteil wird die Reaktion in Gegenwart eines Wasserstoffdonors wie z. B. in Gegenwart von alkylsubstituierten Aromaten, vorzugsweise von Toluol durchgeführt.
Nichtumgesetztes Ausgangsprodukt kann zurückgeführt werden.
Das Malonsäuredinitril kann aus dem Reaktionsprodukt z.B. durch Extraktion mit einem Kohlenwasserstoff und Wasser erhalten werden, wobei die wässrige Phase mit Kochsalz gesättigt wird und das Malonsäuredinitril mit einem Ether reextrahiert wird.

Die 2-Cyano-*N*-alkoxy-acetimidoylhalogenide der allgemeinen Formel 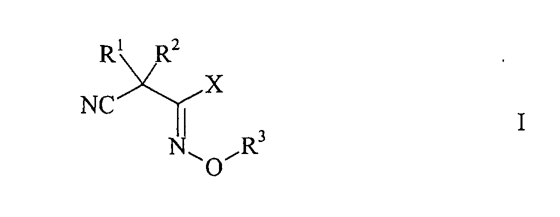 worin R¹, R² und R³ die genannte Bedeutung haben, sind bisher nicht literaturbekannt und damit ebenfalls Gegenstand der Erfindung.
Bevorzugte 2-Cyano-*N*-alkoxy-acetimidoylhalogenide sind das 2-Cyano-*N*-methoxyacetimidoylchlorid und das 2-Cyano-*N*-ethoxy-acetimidoylchlorid.

### Beispiel 1a:

### Herstellung von 2-Cyano-N-methoxy-acetimidoylchlorid

In 200 ml Chloroform wurden bei Raumtemperatur 13,8 g (119,7 mmol) 2-Cyano-*N*-methoxy-acetamid vorgelegt. Die Lösung wurde auf 3 °C gekühlt, dann wurden 29,6 g (139 mmol) PCl₅ in 70 ml Chloroform vorsichtig zugegeben. Nach Abklingen der Gasentwicklung wurde vorsichtig bei 5 °C mit 90 ml Wasser versetzt. Die wässerige Phase wurde abgetrennt und noch zweimal mit 50 ml Methylenchlorid ausgezogen. Die vereinigten organischen Phasen wurden mit NaHCO₃ neutral gewaschen, getrocknet und eingeengt. Der braune Rückstand (11,34 g) wurde zur weiteren Reinigung einer Destillation bei 85 °C / 10 mbar unterzogen. Man erhielt 8,5 g (53 %) einer farblosen Flüssigkeit, welche nach ¹H-NMR rein war.

| | |
|---|---|
| ¹H-NMR (400 MHz, CDCl₃) δ | 3,60 (s, 2H, CH₂); |
| | 4,01 (s, 3H, OCH₃). |
| | |
| ¹³C-NMR (400 MHz, CDCl₃) δ | 128,3 (s); |
| | 113 (s); |
| | 63,4 (q); |
| | 26,3 (t). |

### Beispiel 1b:

### Herstellung von 2-Cyano-N-ethoxy-acetimidoylchlorid

Zu einer Lösung von 12,0 g (123,0 mmol) *O*-Ethylhydroxylamin-Hydrochlorid und 11,61 g (117,2 mmol) Cyanessigsäuremethylester in 100 ml Methanol wurde bei Raumtemperatur langsam 13,69 g (135,3 mmol) Triethylamin zugetropft und das resultierende Gemisch wurde 60 Stunden bei Raumtemperatur gerührt. Obwohl der Umsatz noch nicht vollständig war, wurde das Reaktionsgemisch bis zur Trockene eingeengt. Flash-Säulenchromatographie (Silicagel, zuerst Essigester/Hexan 1:1, dann Essigester) des Rückstandes lieferte 8,20 g (55 %) des Titelproduktes als weissen Feststoff.

| | |
|---|---|
| ¹H-NMR (400 MHz, DMSO-*d*₆) δ | 11,2 (s, breit, NH); |
| | 3,80 (q, 2H); |
| | 3,55 (s, 2H); |
| | 1,15 (t, 3H). |
| | |
| ¹³C-NMR (400 MHz, DMSO-*d*₆) δ | 159,37 (C=O); |
| | 115,53 (C≡N); |
| | 70,88 (OCH₂); |
| | 22,89 (CH₂); |
| | 13,27 (CH₃). |

### Beispiel 1c:

### Herstellung von 2-Cyano-N-ethoxy-acetimidoylchlorid

Zu einer Lösung von 3,90 g (0,030 mol) 2-Cyano-*N*-ethoxy-acetamid in 70 ml Chloroform wurde bei 3 °C eine Suspension von 7,60 g (0,037 mmol) Phosphorpentachlorid in 30 ml Chloroform langsam zugetropft. Das leicht trübe Reaktionsgemisch wurde eine Stunde bei Raumtemperatur gerührt. Unter Eiskühlung wurden 40 ml H₂O zugetropft. Die Phasen wurden getrennt und die wässrige Phase wurde mit Chloroform (2 × 50 ml) extrahiert. Die vereinigten org. Phasen wurden mit einer Na₂CO₃-Lösung (pH ≈ 11; 2 × 20 ml) gewaschen, mit Na₂SO₄ getrocknet und das Lösungsmittel wurde am Rotavap abdestilliert. Kugelrohrdestillation (2 - 4 mbar, 150 °C Ofentemperatur) des Rückstandes lieferte 3,21 g (72%) des Titelproduktes als klares, farbloses Öl.

| | |
|---|---|
| ¹H-NMR (400 MHz, CDCl₃) δ | 4,25 (q, 2H); |
| | 3,60 (s, 2H); |
| | 1,32 (t, 3H). |
| | |
| ¹³C-NMR (400 MHz, CDCl₃) δ | 127,78 (Cl-C=N); |
| | 113,46 (C≡N) ; |
| | 71,62 (OCH₂); |
| | 26,41 (CH₂); |
| | 14,36 (CH₃). |

### Beispiel 2a:

### Herstellung von Malonsäuredinitril

129 mg 2-Cyano-*N*-methoxy-acetimidoylchlorid wurden in 10 ml Toluol gelöst. Diese Lösung wurde portionsweise, aufgeteilt in 100 µl Portionen, während 45 Minuten so in eine Verdampfungskugel, die mit einem auf 870 °C erwärmten Quarz-Pyrolyserohr (Länge 30 cm, Innendurchmesser 2,5 cm) verbunden war, eingespritzt, dass der durch die Vakuumpumpe reduzierte Druck auf 0,2 mbar gehalten werden konnte.
Die Reaktionsprodukte wurden in einer auf -196 °C gekühlten Kühlfalle aufgefangen. Die Analyse des Reaktionsgemisches mittels ¹H-NMR und GC-MS zeigte neben unreagiertem Ausgangsprodukt und Bibenzyl eine Ausbeute an Malonsäuredinitril von 27% bezogen auf eingesetztes Ausgangsprodukt.

### Beispiel 2b:

### Herstellung von Malonsäuredinitril

146 mg 2-Cyano-*N*-ethoxy-acetimidoylchlorid wurden in 10 ml Toluol gelöst. Diese Lösung wurde portionsweise, aufgeteilt in 100 µl Portionen, während 45 Minuten so in eine Verdampfungskugel, die mit einem auf 870 °C erwärmten Quarz-Pyrolyserohr (Länge 30 cm, Innendurchmesser 2,5 cm) verbunden war, eingespritzt, dass der durch die Vakuumpumpe reduzierte Druck auf 0,2 mbar gehalten werden konnte.
Die Reaktionsprodukte wurden in einer auf-196 °C gekühlten Kühlfalle aufgefangen. Die Analyse des Reaktionsgemisches mittels ¹H-NMR und GC-MS zeigte neben unreagiertem Ausgangsprodukt und Bibenzyl eine Ausbeute an Malonsäuredinitril von 25% bezogen auf eingesetztes Ausgangsprodukt.

### Beispiel 3:

### Herstellung und Reinigung von Malonsäuredinitril

510 mg 2-Cyano-*N*-methoxy-acetimidoylchlorid wurden in 50 ml Toluol gelöst. Diese Lösung wurde portionsweise, aufgeteilt in 100 µl Portionen, während 130 Minuten so in eine Verdampfungskugel, die mit einem auf 870 °C erwärmten Quarz-Pyrolyserohr (Länge 30 cm, Innendurchmesser 2,5 cm) verbunden war, eingespritzt, dass der durch die Vakuumpumpe reduzierte Druck auf 0,3 mbar gehalten werden konnte.
Die Reaktionsprodukte wurden in einer auf-196 °C gekühlten Kühlfalle aufgefangen.

Der Kühlfalleninhalt wurde in einen Scheidetrichter gegeben und die Kühlfalle wurde mit 50 ml Hexan und zweimal mit 40 ml Wasser gespült. Die vereinigten Lösungen wurden mit Wasser (insgesamt 150 ml) extrahiert und die Phasen getrennt.

Die wässrige Phase wurde mit 52 g Kochsalz versetzt und anschliessend dreimal mit Diethylether (insgesamt 700 ml) ausgeschüttelt. Die etherische Phase wurde mit MgSO₄ getrocknet und das Lösungsmittel im Vakuum entfernt. Der Rückstand bestand laut ¹H-NMR zu 95% aus Malonsäuredinitril und zu 5% aus dem Ausgangsprodukt. Die Ausbeute betrug 26% bezogen auf eingesetztes Ausgangsprodukt.

Analog zu Beispiel 2, lediglich mit unterschiedlicher Temperatur des Quarzrohrs, wurden nachfolgende Versuche durchgeführt.

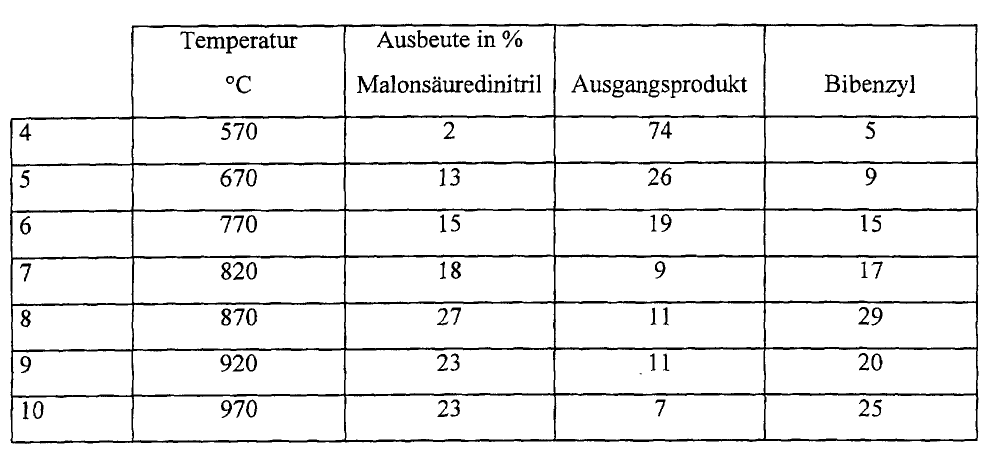

## Patentansprüche

1. Verfahren zur Herstellung von Malonsäuredinitril und substituierten Malonsäuredinitrilen, **dadurch gekennzeichnet, dass** ein 2-Cyano-*N*-alkoxy-acetimidoylhalogenid der allgemeinen Formel 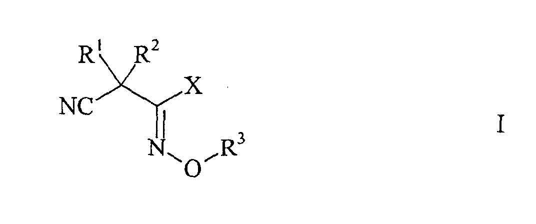 worin
R¹ und R² gleich oder verschieden sind und Wasserstoff, C₁₋₆-Alkyl oder durch C₁₋₄Alkyl, C₁₋₄-Alkoxy, C₁₋₄-Alkanoyl, Halogen, Nitro, Amino, Alkylamino oder Dialkylamino substituiertes C₁₋₆-Alkyl bedeuten;
R³ C₁₋₆-Alkyl, durch C₁₋₄-Alkyl, C₁₋₄-Alkoxy, C₁₋₄-Alkanoyl, Halogen, Nitro, Amino, Alkylamino oder Dialkylamino substituiertes C₁₋₆-Alkyl, Cyclo-C₃₋₆-alkyl, Phenyl, durch C₁₋₄-Alkyl, C₁₋₄-Alkoxy, C₁₋₄-Alkanoyl, Halogen, Nitro, Amino, Alkylamino oder Dialkylamino substituiertes Phenyl, Naphthyl, Benzyl, oder eine Gruppe der Formel 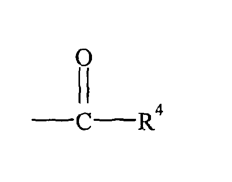 bedeutet, worin
R⁴ C₁₋₆-Alkyl oder durch C₁₋₄-Alkyl, C₁₋₄-Alkoxy, C₁₋₄-Alkanoyl, Halogen, Nitro, Amino, Alkylamino oder Dialkylamino substituiertes C₁₋₆-Alkyl, Phenyl, durch C₁₋₄-Alkyl, C₁₋₄-Alkoxy, C₁₋₄-Alkanoyl, Halogen, Nitro, Amino, Alkylamino oder Dialkylamino substituiertes Phenyl, Naphthyl oder Benzyl bedeutet; und
X ein Halogenatom bedeutet;
bei einer Temperatur von 500 °C bis 1000 °C umgesetzt wird.

2. Verfahren nach Patentanspruch 1, **dadurch gekennzeichnet, dass** die Umsetzung bei einer Temperatur von 700 °C bis 1000 °C durchgeführt wird.

3. Verfahren nach Patentanspruch 1 oder 2, **dadurch gekennzeichnet, dass** das 2-Cyano-*N*-alkoxy-acetimidoylhalogenid der allgemeinen Formel I durch Halogenierung eines 2-Cyano-*N*-alkoxy-acetamids der allgemeinen Formel 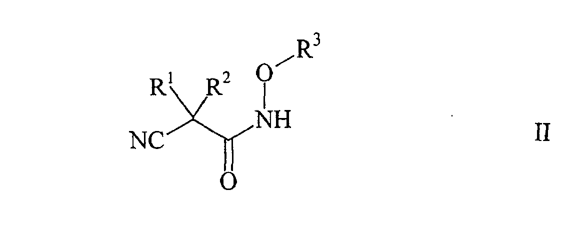 worin R¹, R² und R³ die genannte Bedeutung haben, hergestellt wird.

4. Verfahren nach Patentanspruch 3, **dadurch gekennzeichnet, dass** die Halogenierung eine Chlorierung ist und mit Phosphorpentachlorid, Phosgen, Phosphoroxychlorid oder Tetrachlorkohlenstoff und Triphenylphosphin durchgeführt wird.

5. Verfahren nach Patentanspruch 3 oder 4, **dadurch gekennzeichnet, dass** die Halogenierung in Gegenwart eines halogenierten Lösungsmittels bei einer Reaktionstemperatur von -20 °C bis 150 °C durchgeführt wird.

6. 2-Cyano-*N*-alkoxy-acetimidoylhalogenid der allgemeinen Formel 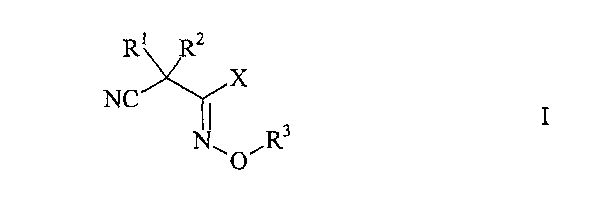 worin R¹, R², R³ und X die in Anspruch 1 angegebene Bedeutung haben.

## Claims

1. Process for the preparation of malononitrile and substituted malononitriles, **characterized in that** a 2-cyano-*N*-alkoxyacetimidoyl halide of the general formula 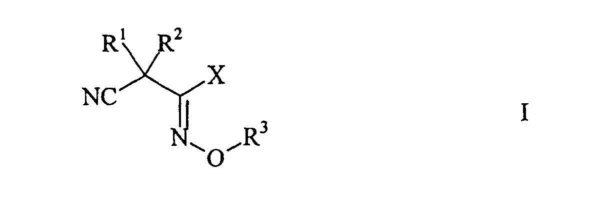 wherein
R¹ and R² are identical or different and are hydrogen, C₁₋₆-alkyl, or C₁₋₆ alkyl substituted with C₁₋₄-alkyl, C₁₋₄-alkoxy, C₁₋₄-alkanoyl, halogen, nitro, amino, alkylamino or dialkylamino,
R³ is C₁₋₆-alkyl, C₁₋₆-alkyl substituted with C₁₋₄-alkyl, C₁₋₄-alkoxy, C₁₋₄-alkanoyl, halogen, nitro, amino, alkylamino or dialkylamino, cyclo-C₃₋₆-alkyl, phenyl, phenyl substituted with C₁₋₄-alkyl, C₁₋₄-alkoxy, C₁₋₄-alkanoyl, halogen, nitro, amino, alkylamino or dialkylamino, naphthyl, benzyl, or a group of the formula 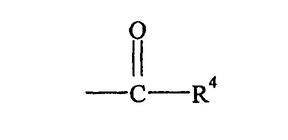 wherein
R⁴ is C₁₋₆-alkyl, C₁₋₆-alkyl substituted with C₁₋₄-alkyl, C₁₋₄-alkoxy, C₁₋₄-alkanoyl, halogen, nitro, amino, alkylamino or dialkylamino, phenyl, phenyl substituted with C₁₋₄-alkyl, C₁₋₄-alkoxy, C₁₋₄-alkanoyl, halogen, nitro, amino, alkylamino or dialkylamino, naphthyl or benzyl, and
X is a halogen atom,
is converted at a temperature of from 500°C to 1000°C.

2. Process according to Claim 1, **characterized in that** the conversion is carried out at a temperature of from 700°C to 1000°C.

3. Process according to Claim 1 or 2, **characterized in that** the 2-cyano-*N*-alkoxyacetimidoyl halide of the general formula I is prepared by halogenation of a 2-cyano-*N*-alkoxyacetamide of the general formula 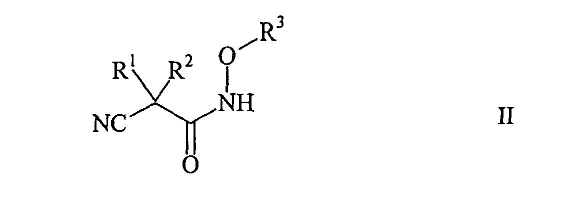 wherein R¹, R² and R³ are as defined above.

4. Process according to Claim 3, **characterized in that** the halogenation is a chlorination and is carried out using phosphorus pentachloride, phosgene, phosphorus oxychloride or tetrachloromethane and triphenylphosphine.

5. Process according to Claim 3 or 4, **characterized in that** the halogenation is carried out in the presence of a halogenated solvent at a reaction temperature of from -20°C to 150°C.

6. 2-Cyano-*N*-alkoxyacetimidoyl halide of the general formula 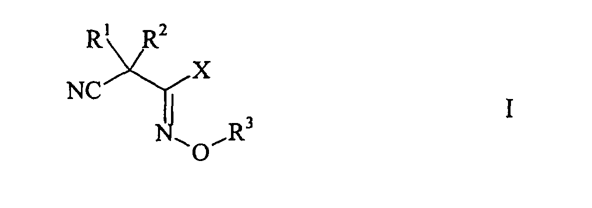 wherein R¹, R², R³ and X are as defined in Claim 1.

## Revendications

1. Procédé pour la préparation du malonitrile et de malonitriles substitués, **caractérisé en ce qu'**on convertit un halogénure de 2-cyano-*N*-alcoxy-acétimidoyle de formule générale 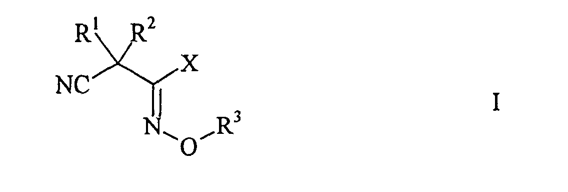 dans laquelle
R¹ et R² sont identiques ou différents et représentent un atome d'hydrogène, un groupe alkyle en C₁₋₆ ou un groupe alkyle en C₁₋₆ substitué par un groupe alkyle en C₁₋₄, alcoxy en C₁₋₄, alcanoyle en C₁₋₄ ou par un atome d'halogène ou par un groupe nitro, amino, alkylamino ou dialkylamino ;
R³ représente un groupe alkyle en C₁₋₆ ou un groupe alkyle en C₁₋₆ substitué par un groupe alkyle en C₁₋₄, alcoxy en C₁₋₄, alcanoyle en C₁₋₄ ou par un atome d'halogène ou par un groupe nitro, amino, alkylamino ou dialkylamino, un groupe cycloalkyle en C₃₋₆, phényle, phényle substitué par un groupe alkyle en C₁₋₄, alcoxy en C₁₋₄, alcanoyle en C₁₋₄ ou par un atome d'halogène ou par un groupe nitro, amino, alkylamino ou dialkylamino, le groupe phényle, naphthyle, benzyle ou un groupe de formule 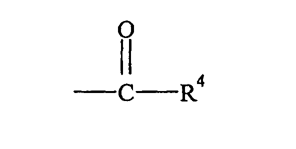 dans laquelle
R⁴ représente un groupe alkyle en C₁₋₆ ou un groupe alkyle en C₁₋₆ substitué par un groupe alkyle en C₁₋₄, alcoxy en C₁₋₄, alcanoyle en C₁₋₄ ou par un atome d'halogène ou par un groupe nitro, amino, alkylamino ou dialkylamino, le groupe phényle, un groupe phényle substitué par un groupe alkyle en C₁₋₄, alcoxy en C₁₋₄, alcanoyle en C₁₋₄ ou par un atome d'halogène ou par un groupe nitro, amino, alkylamino ou dialkylamino, le groupe phényle, naphthyle ou benzyle ; et
X représente un atome d'halogène,
à une température de 500°C à 1000°C.

2. Procédé selon la revendication 1, **caractérisé en ce qu'**on effectue la réaction à une température de 700°C à 1000°C.

3. Procédé selon la revendication 1 ou 2, **caractérisé en ce que** l'halogénure de 2-cyano-*N*-alcoxy-acétimidoyle de formule générale I est préparé par halogénation d'un 2-cyano-*N*-alcoxy-acétamide de formule générale 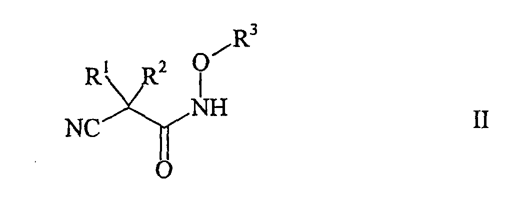 dans laquelle R¹, R² et R³ ont les significations données.

4. Procédé selon la revendication 3, **caractérisé en ce que** l'halogénation est une chloration et est effectuée avec du pentachlorure de phosphore, du phosgène, de l'oxychlorure de phosphore ou du tétrachlorure de carbone et de la triphénylphosphine.

5. Procédé selon la revendication 3 ou 4, **caractérisé en ce que** l'halogénation est effectuée en présence d'un solvant halogéné, à une température de réaction de -20°C à 150°C.

6. Halogénure de 2-cyano-*N*-alcoxy-acétimidoyle de formule générale 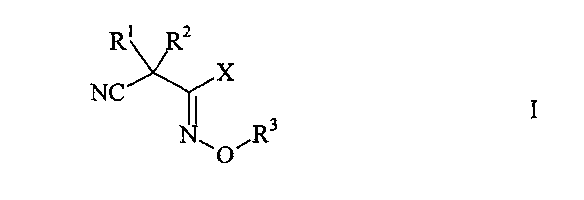 dans laquelle R¹, R², R³ et X ont les significations données dans la revendication 1.
